# FASCICULE DE BREVET EUROPEEN

(11) **EP 0 968 704 B1**
(45) Date de publication et mention de la délivrance du brevet: **06.12.2000**
(21) Numéro de dépôt: 99401436.3
(22) Date de dépôt: 11.06.1999
(51) Int. Cl.: A61K 7/00, A61K 9/127

(54) **Composition cosmétique ou dermatologique**
Kosmetische oder dermatologische Zubereitung
Cosmetic or dermatological composition

(30) Priorité: 03.07.1998 FR 9808559
(43) Date de publication de la demande: 05.01.2000
(73) Titulaire: L'OREAL, 75008 Paris (FR)
(72) Inventeur: Biatry, Bruno, 94300 Vincennes (FR)
(74) Mandataire: Rasson, Catherine

(56) Documents cités:
- EP-A- 0 711 540
- WO-A-95/34287
- J. SEDDON: "an inverse face-centered cubic phase formed by diacylglycerol-phosphatidylcholine mixtures" BIOCHEMISTRY, vol. 29, no. 34, 1990, pages 7997-8002, XP000612108
- M. LEAVER ET AL.: "phase behaviour and structure in a non-ionic surfactant-oil-water mixture" J. CHEM. SOC., FARADAY TRANS., vol. 91, no. 23, 1995, pages 4269-4274, XP002100883
- K. LARSSON: "the structure of mesomorphic phases and micelles in aqueous glyceride systems" ZEITSCHRIFT FÜR PHYSIKALISCHE CHEMIE NEUE FOLGE, vol. 56, no. 3-4, 1967, pages 173-198, XP002100884

## Description

La présente invention a pour objet une composition se présentant sous forme d'une dispersion d'une phase huileuse et d'une phase aqueuse, la dispersion étant stabilisée à l'aide de particules de gel cubique formées à l'aide d'une association de deux composés amphiphiles, ainsi que l'utilisation de la dite composition, notamment dans les domaines cosmétique, dermatologique et/ou pharmaceutique, plus particulièrement pour le soin et/ou le conditionnement et/ou l'hygiène et/ou le maquillage de la peau, des muqueuses, du cuir chevelu et/ou des cheveux.

Une grande variété de produits se présente sous forme d'une dispersion d'une phase huileuse et d'une phase aqueuse, la phase interne pouvant être aqueuse (émulsion eau-dans-huile ou E/H) ou huileuse (émulsion huile-dans-eau ou H/E). Il s'agit tout particulièrement de produits topiques cosmétiques, dermatologiques ou pharmaceutiques, ces dispersions conférant à la peau de bonnes propriétés sensorielles. Toutefois, il est bien connu que ces dispersions manquent de stabilité dans le temps et "cassent", donnant naissance à deux phases distinctes les rendant inutilisables. Pour stabiliser ces dispersions, on y ajoute de manière habituelle un émulsionnant ; la nature et la concentration de l'émulsionnant utilisé peut influer de façon significative sur la stabilité de telles compositions. En outre, il est bien certain que le choix et la concentration d'un émulsionnant approprié va dépendre de divers facteurs et en particulier de l'huile ou des huiles constituant la phase huileuse de la dispersion ou de l'émulsion.

Par ailleurs, certains émulsionnants ne sont pas sans inconvénients, notamment lorsqu'ils sont employés à forte concentration en vue d'améliorer la stabilité de la dispersion. En effet, ils peuvent provoquer certains phénomènes d'irritation, en particulier sur les peaux sensibles.

Pour pallier à cet inconvénient, il a été proposé dans le document EP-A-711540 de stabiliser les dispersions H/E à l'aide de particules de gel cubique.

Le terme de gel cubique désigne des gels transparents, isotropes en lumière polarisée, se présentant sous forme de phase cristal liquide cubique. Les phases cubiques sont organisées d'une manière bi-polaire en domaines hydrophile et lipophile distincts, en contact étroit et formant un réseau tridimensionnel thermodynamiquement stable. Une telle organisation a été notamment décrite dans "La Recherche", Vol.23, pp.306-315, Mars 1992 et dans "Lipid Technology", Vol.2, n°2, pp.42-45, Avril 1990. Selon l'arrangement des domaines hydrophile et lipophile, la phase cubique est dite de type normal ou inverse. Le terme de gel cubique utilisé selon la présente invention regroupe bien entendu les gels présentant les différents types de phases cubiques.

Toutefois, la technique décrite dans le document EP-A-711540 présente l'inconvénient de permettre uniquement l'obtention d'émulsions H/E.

Il a été maintenant trouvé de manière surprenante que l'on pouvait obtenir des dispersions ou émulsions stables aussi bien E/H que H/E, en utilisant des particules de gel cubique, obtenues à partir d'un mélange de deux composés amphiphiles susceptibles de réagir de manière différente en présence d'eau : l'un des composés amphiphiles est susceptible de former une phase lamellaire et l'autre est susceptible de former une phase hexagonale inverse.

Ainsi, la présente invention a pour objet une composition sous forme d'une dispersion comprenant une phase aqueuse et une phase huileuse, caractérisée en ce qu'elle comprend en outre des particules de gel cubique, formées par un mélange d'au moins deux composés amphiphiles, l'un des composés amphiphiles étant susceptible de former en présence d'eau une phase lamellaire et l'autre étant susceptible de former en présence d'eau une phase hexagonale inverse.

Le mélange des deux composés amphiphiles formant les particules de gel cubique est caractérisé par le fait qu'aucun des deux composés amphiphiles ne peut conduire par lui-même à une phase cubique lorsque qu'il est mis en présence d'eau et que seul leur mélange conduit à une telle phase, et que, par ailleurs, l'un des composés amphiphiles est susceptible de former en présence d'eau une phase lamellaire, tandis que l'autre composé amphiphile est susceptible de former en présence d'eau une phase hexagonale inverse.

On entend par phase lamellaire (phase D selon EKWALL), une phase cristal liquide à symétrie plane, comprenant plusieurs bicouches d'amphiphile disposées en parallèle et séparées par un milieu liquide qui est généralement de l'eau.

On entend par phase hexagonale inverse (phase F selon EKWALL), une phase cristal liquide correspondant à une arrangement hexagonal de cylindres parallèles remplis d'un milieu liquide qui est généralement de l'eau, séparés par un environnement hydrocarboné correspondant aux chaînes grasses de l'amphiphile.

Une description plus précise de ces phases peut être trouvée dans la Revue Française des Corps Gras, n°2, Février 1969, p 87 à 111, (Lachampt et Vila, « Textures des phases paracristallines »).

Le composé amphiphile susceptible de former une phase lamellaire est choisi parmi l'isostéarate de diglycérol (Solvay) et le mono-oléate de diglycérol (RYLO PG 29^{R} commercialisé par la société Danisco), seuls ou en mélange.

Le composé amphiphile susceptible de former une phase hexagonale inverse est choisi parmi le décyl-2 tétradécanoate de diglycérol, le di/tri-oléate de diglycérol (TSED 396^{R} commercialisé par la société Danisco), le 3-N-(2-décyltétradécyloxycarbonyl)-amino-1,2-propanediol et le N-2-dodécylhexadécyloxycarbonyl-N-méthyl-D-glucamine et leurs mélanges. Ces carbamates d'aminopolyols sont décrits dans le document EP-A-666251 cité ici pour référence.

De préférence, le mélange des deux types de composés amphiphiles est constitué de 10 à 90 % en poids et mieux 15 à 85 % en poids d'au moins un composé amphiphile susceptible de former une phase lamellaire et de 10 à 90 % en poids et mieux de 15 à 85 % en poids d'au moins un composé amphiphile susceptible de former une phase hexagonale inverse, par rapport au poids total du mélange.

Le rapport entre les deux types de composés amphiphiles dépend des composés utilisés et l'homme du métier saura déterminer la quantité de chaque type de composé à utiliser afin d'obtenir des particules de gels cubiques.

Plus précisément, les mélanges constituant les particules de gel cubique des compositions de l'invention sont de préférence réalisés à l'aide des associations suivantes :
- 55 à 75 % en poids d'isostéarate de diglycérol et 25 à 45 % en poids de décyl-2 tétradécanoate de diglycérol ;
- 30 à 65 % en poids d'isostéarate de diglycérol et 35 à 70 % en poids de di/tri-oléate de diglycérol ;
- 75 à 85 % en poids d'isostéarate de diglycérol et 15 à 25 % en poids de 3-N-(2-décyl-tétradécyloxycarbonyl)-amino-1,2-propanediol ;
- 55 à 75 % en poids d'isostéarate de diglycérol et 25 à 45 % en poids de N-2-dodécylhexadécyl-oxycarbonyl-N-méthyl-D-glucamine ;
- 15 à 50 % en poids de mono-oléate de diglycérol et 50 à 85 % en poids de diltri-oléate de diglycérol.

La composition comprend de préférence de 0,1 à 15 % en poids et mieux de 0,5 à 10 % en poids de composés amphiphiles constituant les particules de la phase cubique par rapport au poids total de la composition et/ou de 2 à 40 % en poids de phase huileuse par rapport au poids total de la composition.

Le rapport en poids des composés amphiphiles constituant les particules de la phase cubique et de la phase huileuse va de préférence de 0,02/1 à 1/1, et mieux de 0,05/1 à 0,5/1.

Dans la composition selon l'invention, les particules de gel cubique ont une taille allant de préférence de 0,05 µm à 1 µm.

Quand la composition est une émulsion H/E, la taille des gouttelettes de la phase huileuse dispersée dans la phase aqueuse à l'aide de ces particules va de préférence de 0,1 à 10 µm.

La composition selon l'invention peut contenir en outre un agent dispersant et stabilisant choisi parmi les tensioactifs hydrosolubles à température ambiante, contenant une chaîne grasse, saturée ou insaturée, linéaire ou ramifiée, ayant de 8 à 22 atomes de carbone. Cet agent est de préférence présent en une quantité allant de 0,1 à 3 % du poids total de la composition.

L'agent dispersant et stabilisant tel que défini précédemment est de préférence choisi parmi :
(1) les alkyl ou alcényl éthers ou esters de polyol ;
(2) les amino-acides N-acylés et leurs dérivés, ainsi que les peptides N-acylés par un radical alkyle ou alcényle et leurs sels ;
(3) les alkyl ou alcényl éthers ou esters sulfates, leurs dérivés et leurs sels ;
(4) les aikyl ou alcényl éthers ou esters gras polyoxyéthylénés ;
(5) les acides alkyl ou alcényl carboxyliques polyoxyéthylénés et leurs sels ;
(6) les N-alkyl ou alcényl bétaïnes ;
(7) les alkyl ou alcényl triméthylammonium et leurs sels, et
(8) leurs mélanges.

Dans les composés ci-dessus énumérés, les radicaux alkyle et alcényle ont de préférence de 8 à 22 atomes de carbone et peuvent se présenter sous forme de mélanges.

### 1 - Alkyl ou alcényl éthers ou esters de polyol

Parmi ceux-ci, on peut citer notamment les alkyl ou alcényl esters de sorbitan polyoxyéthylénés comportant au moins 20 motifs d'oxyde d'éthylène, tels que le palmitate de sorbitan 20 OE ou Polysorbate 40 commercialisé sous la dénomination de Montanox 40 DF^{R} par la Société Seppic, et le laurate de sorbitan 20 OE ou Polysorbate 20 commercialisé sous la dénomination de Tween 20^{R} par la société ICI.

Dans ce groupe, on peut également citer les alkyl ou alcényl esters de polyglycérol comportant au moins 10 motifs dérivés du glycérol, oxyéthylénés ou non, tels que le polyglycéryl-10 laurate commercialisé sous la dénomination de Decaglyn 1-L^{R} par la société Nikko Chemicals.

On peut encore citer les alkyl ou alcényl éthers ou esters de mono ou polysaccharides tels que ceux dérivant du glucose, du fructose, du galactose, du maltose ou du lactose et notamment les monoesters en 1- et 6- du D-fructose, du décylglucose et du décylpolyglucose.

### 2 - Amino-acides N-acylés et leurs dérivés, les peptides N-acylés par un radical alkyle ou alcényle et leurs sels

Parmi ceux-ci, on utilise de préférence ceux pour lesquels le radical alkyle ou alcényle a au moins 12 atomes de carbone. Par amino-acides, on entend selon l'invention les α, β, γ-amino-acides. Comme sels d'amino-acides N-acylés, on peut citer par exemple ceux de N-acyl glutamate, tels que le cocoylglutamate de monosodium, le lauroylglutamate de monosodium, l'alcoyl-C₁₄-C₂₀-glutamate de disodium (le radical alcoyle C₁₄-C₂₀ dérivant du suif hydrogéné), commercialisés respectivement sous les dénominations de Acylglutamate CS-11^{R}, Acylglutamate LS-11^{R} et de Acylglutamate HS-21^{R} par la société Ajinomoto.

On peut encore citer les lysines N-acylées telles que la lauroyllysine commercialisée sous la dénomination de Amihope LL^{R} par la société Ajinomoto.

Les dérivés d'amino-acides N-acylés et leurs sels sont de préférence les sarcosinates N-acylés tels que le lauroylsarcosinate de sodium commercialisé sous la dénomination de Oramix L30^{R} par la société Seppic, le myristoylsarcosinate de sodium et le palmitoyl-sarcosinate de sodium commercialisés respectivement sous les dénominations de Nikkol Sarcosinate MN^{R} et de Nikkol Sarcosinate PN^{R} par la société Nikko Chemicals.

Parmi les peptides N-acylés on peut citer ceux dérivés de tout ou partie du collagène ou de la kératine tels que le lauroylcollagène de sodium et la palmitoylkératine commercialisés respectivement sous les dénominations de Proteol B 30^{R} et de Lipacide PK^{R} par la société Seppic.

### 3 - Alkyl ou alcényl éthers ou esters sulfates, leurs dérivés et leurs sels

Parmi ceux-ci, on utilise de préférence ceux pour lesquels le radical alkyle ou alcényle a au moins 12 atomes de carbone. Parmi les alkyl ou alcényl éthers sulfates, on utilise de préférence les sels d'alkyl éther sulfate et notamment le lauryléther sulfate de sodium.

Parmi les alkyl ou alcényl esters sulfates, on peut citer par exemple les esters de l'acide iséthionique ainsi que ses sels et notamment le cocoyl-iséthionate de sodium commercialisé sous la d,nomination de Geropon AC 78^{R} par la société Rhône Poulenc.

### 4 - Alkyl ou alcényl éthers ou esters gras polyoxyéthylénés

Parmi ceux-ci, on utilise de préférence ceux pour lesquels le radical alkyle ou alcényle a au moins 12 atomes de carbone. Ceux particulièrement préférés ont au moins 20 motifs d'oxyde d'éthylène tels que par exemple le PEG-20 stéarate, le laureth-23, l'oleth-20 et le PEG-25 phytostérol.

### 5 - Acides alkyl ou alcényl carboxyliques polyoxyéthylénés et leurs sels

Parmi ceux-ci, on utilise de préférence ceux comportant au moins 10 motifs d'oxyde d'éthylène, tels que par exemple l'acide laureth-10 carboxylique et l'acide oleth-10 carboxylique.

### 6 - N-alkyl ou alcényl bétaïnes

Parmi celles-ci, on utilise de préférence celles pour lesquelles le radical alkyle ou alcényle a au moins 12 atomes de carbone, telles que par exemple la laurylamidopropyl-bétaïne et l'oléylamidopropyl-bétaïne.

### 7 - Alkyl ou alcényl triméthylammonium et leurs sels

Parmi ceux-ci, on utilise de préférence ceux pour lesquels le radical alkyle ou alcényle a au moins 12 atomes de carbone. Comme sels, on utilise de préférence les bromures et chlorures tels que le chlorure de cocoyltriméthylammonium et le bromure de cétyltriméthylammonium.

Selon un mode de réalisation particulier de l'invention, la composition selon l'invention comprend en outre de 0,0005 % à 5 % en poids et de préférence de 0,001 % à 2 % en poids d'au moins un lipide amphiphile ionique non hydrosoluble, par rapport au poids total de la composition.

Comme lipide amphiphile ionique non hydrosoluble, on peut notamment citer :
(i) les phopholipides tels que les phospholipides naturels comme la lécithine de soja ou d'oeuf, les phospholipides modifiés par voie chimique ou enzymatique comme la lécithine hydrogénée ou le sel de sodium de l'acide phosphatidique, et les phospholipides de synthèse comme la dipalmitoylphosphatidylcholine ;
(ii) les esters phosphoriques d'alcool gras tels que le monocétyl phosphate et ses sels de sodium et de potassium, commercialisé sous la dénomination de Monafax 160^{R} par la société Mona, ainsi que le dimyristyl phosphate et ses sels de sodium et de potassium, commercialisé sous la dénomination de Mexoryl SY^{R} par la société Chimex ;
(iii) les dérivés N-acylés de l'acide glutamique tels que le stéaroyl glutamate de monosodium commercialisé sous la dénomination de Acylglutamate HS 11^{R} par la société Ajinomoto et le mélange cocoyl-alcoyl-C₁₄-C₂₀-glutamate de monosodium, le radical alcoyle C₁₄-C₂₀ dérivant du suif hydrogéné, commercialisé sous la dénomination de Acylglutamate GS 11^{R} par la société Ajinomoto ;
(iv) le cétylsulfate de sodium commercialisé sous la dénomination de Nikkol SCS^{R} par la société Nikko Chemicals ;
(v) le cocoyl-monoglycéride sulfate de sodium commercialisé sous la dénomination de Nikkol SGC 80 N^{R} par la société Nikko Chemicals ; et
(vi) les dérivés d'ammonium quaternaire, tels que le chlorure de béhényltriméthylammonium, le chlorure de dilauryldiméthylammonium, le chlorure de distéaryldiméthylammonium, le méthylsulfate de 4,5-dihydro 1-méthyl 2-alcoyl-C₁₄-C₂₀-1-(2-alcoyl-C₁₄-C₂₀-aminoéthyl)-imidazolium, les radicaux alcoyles C₁₄-C₂₀ dérivant du suif hydrogéné, commercialisé sous la dénomination Rewoquat W75H^{R} par la société Rewo Chemische, le méthylsulfate de dialkylhydroxyéthylméthylammonium dont les radicaux alkyle dérivent du suif, hydrogéné ou non, commercialisé sous la dénomination de Stepanquat VP 85^{R} par la société Stepan, et le quaternium-82 commercialisé par la société Seppic sous la dénomination de Amonyl DM^{R}.

L'incorporation de ces lipides amphiphiles ioniques non hydrosolubles confère aux particules de gel cubique une charge superficielle entraînant une répulsion électrostatique des particules entre elles.

La phase huileuse des compositions selon l'invention comprend de préférence au moins une huile choisie parmi les huiles d'origine végétale ou animale, les huiles minérales et les huiles de synthèse.

Parmi les huiles d'origine végétale, on peut notamment citer l'huile d'amande d'abricot, l'huile de tournesol, l'huile de maïs, l'huile de soja, l'huile de courge, l'huile de pépins de raisin ou de cassis, l'huile de jojoba, l'huile d'amande douce, l'huile de carthame, l'huile de sésame, l'huile de bourrache, l'huile de noisette, l'huile de macadamia et la fraction liquide du beurre de karité. Comme huiles végétales, on peut également utiliser des huiles essentielles telles que l'huile d'eucalyptus, l'huile de lavandin, l'huile de lavande, l'huile de vétiver, l'huile de litsea cubeba, l'huile de citron, l'huile de santal, l'huile de romarin, l'huile de camomille, l'huile de sariette, l'huile de noix de muscade, l'huile de cannelle, l'huile d'hysope, l'huile de carvi, l'huile d'orange, l'huile de géraniol, l'huile de cade et l'huile de bergamote.

Parmi les huiles d'origine animale, on peut notamment citer les huiles de poisson, l'huile de tortue, l'huile de vison ainsi que le squalène hydrogéné (ou perhydrosqualène).

Comme huiles minérales, on peut notamment citer l'huile de paraffine et les isoparaffines.

Parmi les huiles de synthèse, on peut notamment citer les hydrocarbures tels que l'isohexadécane, le polydécène et le polyisobutène, les alcools gras tels que l'octyldodécanol, l'alcool isostéarylique et l'alcool oléique, les esters tels que les glycérides d'acides gras essentiels, les triglycérides des acides caprique et caprylique et leurs mélanges, et les esters d'alcool gras et d'acide gras linéaire ou ramifié, tels que l'huile de purcellin (octanoate de cétéaryle/myristate d'isopropyle) et le mélange d'heptanoate de stéaryle et d'octanoate de stéaryle commercialisé sous la dénomination Dub Solid^{R} par la société Stéarineries Dubois.

Comme huiles de synthèse, on peut également utiliser dans les compositions selon l'invention, des huiles de silicone de type linéaire comme le polydiméthylsiloxane, de type cyclique comme le cyclopentadiméthylsiloxane et le cyclohexadimethylsiloxane, et de type organomodifié comme le polyphényltriméthyl-siloxane et le polydiméthyl siloxane oxyéthyléné et/ou oxypropyléné. On peut encore citer des huiles fluorées telles que les perfluorodécahydronaphtalènes comme la perfluorodécaline, ainsi que des huiles de type perfluoré, telles que les perfluoropolyméthylisopropyléthers.

L'homme du métier veillera à ne pas introduire dans la composition de l'invention des composés de nature ou en quantité susceptibles d'empêcher la préparation d'une composition telle que définie selon l'invention.

Bien entendu, grâce à la structure particulière des particules de gel cubique, il est possible d'incorporer dans celles-ci à la fois des principes actifs hydrophiles et lipophiles, même si ceux-ci présentent une certaine incompatibilité entre eux. Parmi les différents principes actifs pouvant être incorporés, on peut notamment citer les agents antioxydants ou anti-radicaux libres tels que les complexants et les chélateurs (EDTA, tocophérol et ses esters) ; les agents hydratants ou humectants tels que les polyols (glycérine, sorbitol), l'acide hyaluronique et son sel de sodium ; les filtres UV; les agents kératolytiques tels que l'acide rétinoïque et les hydroxyacides tels que l'acide salicylique et ses dérivés ; les accélérateurs de bronzage tels que la caféine et les dérivés de tyrosine ; les dépigmentants tels que l'acide kojique, la vitamine C et ses dérivés tels que l'ascorbyle phosphate de magnésium, l'arbutine et ses dérivés ; les matières colorantes ; les auto-bronzants tels que la dihydroxyacétone et les indoles ; les liporégulateurs tels que le γ-orizanol, l'extrait de Centella asiatica, la caféine et la théophylline ; les agents antivieillissement et antirides tels que les hydroxyacides et notamment les α-hydroxyacides comme l'acide glycolique, l'acide lactique et leurs dérivés, le rétinol et ses dérivés comme l'acétate, le palmitate et le propionate de rétinol, et les rétinoïdes ; les agents anti-inflammatoires et cicatrisants tels que l'acide 18 β-glycyrrhétinique et ses sels, l'α-bisabolol ; les corticoïdes et l'extrait de Centella asiatica ; les antibactériens et antifongiques ; les insectifuges ; les déodorants ; les antipelliculaires ; les agents antichute des cheveux tels que le nicotinate de méthyle ou d'hexyle et le minoxidil ; les colorants capillaires tels que les bases et les coupleurs d'oxydation, les colorants directs et les colorants auto-oxydables ; les agents réducteurs pour permanentes ; les agents conditionneurs pour peau et cheveux.

On peut utiliser dans les compositions selon l'invention soit des particules de gel cubique ne contenant pas de principes actifs, soit des particules contenant au moins un principe actif hydrophile ou lipophile, soit encore des particules contenant à la fois au moins un principe actif hydrophile et au moins un principe actif lipophile.

Selon un mode de réalisation des compositions selon l'invention, il est également possible d'incorporer au moins un principe actif dans la phase huileuse et/ou dans la phase aqueuse. Ce principe actif peut être notamment choisi parmi les principes actifs tels que définis précédemment.

Les compositions selon l'invention peuvent en outre contenir différents additifs conventionnels. Parmi ceux-ci, on peut notamment citer des agents conservateurs, des parfums, des pigments (TiO₂), des matières colorantes, des charges et des agents gélifiants hydrophiles ou lipophiles.

Parmi les agents gélifiants hydrophiles pouvant être utilisés dans les compositions selon l'invention, on peut citer en particulier les dérivés de cellulose tels que l'hydroxyéthylcellulose et les alkylhydroxyéthylcelluloses telles que la cétylhydroxyéthylcellulose ; les dérivés d'algues tels que le satiagum ; les gommes naturelles telles que l'adragante ou la gomme guar ; les polymères synthétiques tels que les polmyères ou copolymères carboxyvinyliques et en particulier ceux commercialisés sous les dénominations de Carbopol^{R} par la société Goodrich ou de Synthalen^{R} par la société 3V SA. La proportion en agent gélifiant va de préférence de 0,1 à 2 % du poids total de la composition.

De préférence, la composition selon l'invention est obtenue selon un procédé de préparation comprenant au moins deux étapes. La première étape consiste généralement à préparer une dispersion aqueuse de particules de gel cubique telles que définies précédemment, par fragmentation, à l'aide d'un homogénéiseur, d'un gel cubique formé à l'aide d'au moins deux composés tels que définis précédemment et d'eau, en présence éventuellement de lipides amphiphiles ioniques non hydrosolubles et/ou de principes actifs hydrophiles et/ou lipophiles et/ou d'un agent dispersant et stabilisant tels que définis précédemment. L'homogénéiseur peut être du type rotor-stator à fort gradient de cisaillement comme le Virtis^{R} ou le Heidolph Diax 600^{R} ou un homogénéiseur haute pression fonctionnant entre 200 et 1.800 bars environ (20 à 180 MPa).

Il est bien entendu possible d'introduire, à ce stade de la préparation de la dispersion aqueuse des particules de gel cubique, divers additifs et/ou principes actifs dans la phase aqueuse. Après la formation des particules de gel cubique, l'agent dispersant et stabilisant se trouve, généralement, à l'extérieur desdites particules.

La deuxième étape consiste généralement ensuite à ajouter à ladite dispersion obtenue une phase huileuse contenant éventuellement certains additifs et/ou principes actifs lipophiles et à soumettre le mélange à une agitation mécanique qui peut être notamment réalisée à l'aide d'un homogénéiseur du même type que ceux définis ci-dessus.

Divers additifs et/ou principes actifs peuvent être également introduits à ce stade dé la préparation. En particulier, lorsque l'on souhaite préparer une dispersion gélifiée, on ajoute généralement au mélange obtenu après la seconde étape, une solution aqueuse contenant un agent gélifiant.

La composition de l'invention peut être notamment destinée à un usage topique et en particulier constituer une composition cosmétique, dermatologique et/ou pharmaceutique ; elle doit alors contenir un milieu physiologiquement acceptable, c'est-à-dire compatible avec la peau, les tissus, les muqueuses et/ou les cheveux des êtres humains. Cette composition peut être plus ou moins fluide, être blanche ou colorée et avoir l'aspect d'une crème, d'une pommade, d'un lait, d'une lotion, d'un sérum, d'une pâte, d'une mousse.

La composition de l'invention peut être appliquée par voie topique notamment sur le visage, y compris autour des yeux, sur le corps ainsi que sur le cuir et les fibres kératiniques telles que les cheveux et les cils des êtres humains.

Les compositions de l'invention, trouvent leur application dans un grand nombre de traitements cosmétiques et/ou dermatologiques de la peau, des muqueuses, plus spécialement des lèvres, et des cheveux, y compris le cuir chevelu.

Les compositions selon l'invention peuvent par exemple être utilisées sur la peau (du visage ou du corps), les muqueuses, le cuir chevelu et/ou les fibres kératiniques telles que les cheveux et les cils, comme produits de soin et/ou de nettoyage et/ou de conditionnement, comme produits de maquillage par incorporation de charges, de pigments ou de colorants, et comme produits de protection solaire après introduction de pigments, de charges et/ou dé filtres appropriés.

Un autre objet de l'invention consiste en un procédé de traitement cosmétique pour le soin et/ou le nettoyage et/ou le conditionnement et/ou le maquillage et/ou la protection de la peau, des fibres kératiniques, du cuir chevelu et/ou des muqueuses, consistant à appliquer sur la peau, les fibres kératiniques, le cuir chevelu et/ou les muqueuses une composition telle que définie ci-dessus.

Un autre objet de l'invention est l'utilisation de la composition selon l'invention pour la préparation d'une composition thérapeutique destinée au traitement et/ou au soin de la peau, des fibres kératiniques, du cuir chevelu et/ou des muqueuses.

D'autres avantages et caractéristiques de l'invention apparaîtront mieux à la lecture des exemples donnés à titre illustratif et non limitatif.

### Exemple 1 : émulsion H/E

| *Première phase :* | |
|---|---|
| Isostéarate de diglycérol | 1,2 % |
| Di/tri-oléate de diglycérol | 1,8 % |
| Eau déminéralisée | 1,25 % |
| | |
| Glycérine | 3 % |
| Polysorbate 40 (Montanox 40 DF^{R}) | 1 % |
| Eau déminéralisée | 52,75 % |
| | |

| *Deuxième phase :* | |
|---|---|
| Huile d'amande d'abricot | 6 % |
| Perhydrosqualène | 6 % |
| Cyclopentadiméthylsiloxane | 2 % |
| | |

| *Troisième phase :* | |
|---|---|
| Cetylhydroxyethylcellulose (Natrosol Plus Grade 330 CS^{R}) | |
| (Société AQUALON) | 1 % |
| Conservateurs | 0,3 % |
| Eau déminéralisée | 23,7 % |

### Exemple 2 : émulsion E/H

| *Première phase :* | |
|---|---|
| Monoléate de diglycérol | 0,9 % |
| Diltri-oléate de diglycérol | 1,65 % |
| Eau déminéralisée | 1,04 % |
| | |
| Glycérine | 2,5 % |
| Polysorbate 40 (Montanox 40 DF^{R}) | 0,21 % |
| Conservateurs | 0,3 % |
| Eau déminéralisée | 78,3 % |
| | |

| *Deuxième phase :* | |
|---|---|
| Heptanoate de stéaryle/Octanoate de stéaryle | 3,5 % |
| Huile de jojoba | 4,2 % |
| Cyclopentadiméthylsiloxane | 6,2 % |
| Cyclohexadimethylsiloxane | 1,2 % |

### Exemple 3 : émulsion E/H

| *Première phase :* | |
|---|---|
| Monoléate de diglycérol | 0,89 % |
| Di/tri-oléate de diglycérol | 1,65 % |
| Stéaroylglutamate de monosodium | 0,01 % |
| Eau déminéralisée | 1,05 % |
| | |
| Glycérine | 3 % |
| Conservateurs | 0,3 % |
| Eau déminéralisée | 77,8 % |
| | |

| *Deuxième phase :* | |
|---|---|
| Heptanoate de stéaryle/Octanoate de stéaryle | 3,6 % |
| Huile d'amande d'abricot | 4,4 % |
| Cyclopentadiméthylsiloxane | 7,3 % |

### Exemple 4 : émulsion H/E

| *Première phase :* | |
|---|---|
| Isostéarate de diglycéryle | 1,8 % |
| Décyl-2 tétradécanoate de diglycérol | 1,2 % |
| Phosphate de monocétyle | 0,03 % |
| Eau déminéralisée | 1,25 % |
| | |
| Glycérine | 3 % |
| Triéthanolamine | 0,01 % |
| Eau déminéralisée | 53,7 % |
| | |

| *Deuxième phase :* | |
|---|---|
| Perhydrosqualène | 7 % |
| Huile d'amande d'abricot | 7 % |
| | |

| *Troisième phase :* | |
|---|---|
| Carbomer (Carbopol 980 ^{R} fourni par la société GOODRICH) | 0,4 % |
| Triéthanolamine | 0,4 % |
| Conservateurs | 0,3 % |
| Eau déminéralisée | 23,91 % |

## Revendications

1. Composition sous forme d'une dispersion comprenant une phase aqueuse et une phase huileuse, caractérisée en ce qu'elle comprend en outre des particules de gel cubique, formées par un mélange d'au moins deux composés amphiphiles, l'un des composés amphiphiles étant susceptible de former en présence d'eau une phase lamellaire et étant choisi parmi l'isostéarate de diglycérol, le mono-oléate de diglycérol et leurs mélanges, et l'autre étant susceptible de former en présence d'eau une phase hexagonale inverse et étant choisi parmi le décyl-2 tétradécanoate de diglycerol, le di/tri-oléate de diglycérol, le 3-N-(2-décyltétradécyloxycarbonyl)-amino-1,2-propanediol et le N-2-dodécylhexadécyloxycarbonyl-N-méthyl-D-glucamine, et leurs mélanges.

2. Composition selon la revendication précédente, caractérisée en ce que le mélange des deux composés amphiphiles est constitué de 10 à 90 % en poids du composé amphiphile susceptible de former une phase lamellaire et de 10 à 90 % en poids du composé amphiphile susceptible de former une phase hexagonale inverse, par rapport au poids total du mélange.

3. Composition selon la revendication 1 ou 2, caractérisée en ce que le mélange des deux composés amphiphiles est choisi parmi les mélanges suivants :
- 55 à 75 % en poids d'isostéarate de diglycérol et 25 à 45 % en poids de décyl-2 tétradécanoate de diglycérol ;
- 30 à 65 % en poids d'isostéarate de diglycérol et 35 à 70 % en poids de di/tri-oléate de diglycérol;
- 75 à 85 % en poids d'isostéarate de diglycérol et 15 à 25 % en poids de 3-N-(2-décyl-tétradécyloxycarbonyl)-amino-1,2-propanediol ;
- 55 à 75 % en poids d'isostéarate de diglycérol et 25 à 45 % en poids de N-2-dodécylhexadécyl-oxycarbonyl-N-méthyl-D-glucamine ;
- 15 à 50 % en poids de mono-oléate de diglycérol et 50 à 85 % en poids de di/tri-oléate de diglycérol.

4. Composition selon, l'une quelconque des revendications précédentes, caractérisée en ce qu'elle comprend de 0,1 à 15 % en poids de composés amphiphiles constituant les particules de la phase cubique par rapport au poids total de la composition.

5. Composition selon l'une quelconque des revendications précédentes, caractérisée en ce qu'elle comprend de 2 à 40% en poids de phase huileuse par rapport au poids total de la composition.

6. Composition selon l'une quelconque des revendications précédentes, caractérisée en ce que le rapport en poids des composés amphiphiles et de la phase huileuse va de 0,02/1 à 1/1 et mieux de 0,05/1 à 0,5/1.

7. Composition selon l'une quelconque des revendications précédentes, caractérisée en ce que les particules de gel cubique ont une taille allant de 0,05 µm à 1 µm.

8. Composition selon l'une quelconque des revendications précédentes, caractérisée en ce qu'elle est une émulsion H/E et que la taille des gouttelettes de la phase huileuse dispersée va de préférence de 0,1 à 10 µm.

9. Composition selon l'une quelconque des revendications 1 à 7, caractérisée en ce qu'elle est une émulsion E/H.

10. Composition selon l'une quelconque des revendications précédentes, caractérisée en ce qu'elle contient en outre au moins un agent dispersant et stabilisant choisi parmi les tensioactifs hydrosolubles à température ambiante, contenant une chaîne grasse, saturée ou insaturée, linéaire ou ramifiée, ayant de 8 à 22 atomes de carbone.

11. Composition selon la revendication précédente, caractérisée en ce que l'agent dispersant et stabilisant est choisi parmi les alkyl ou alcényl esters de sorbitan polyoxyéthylénés comportant au moins 20 motifs d'oxyde d'éthylène.

12. Composition selon l'une quelconque des revendications précédentes, caractérisée en ce qu'elle contient en outre au moins un lipide amphiphile ionique non hydrosoluble.

13. Composition selon l'une quelconque des revendications précédentes, caractérisée en ce que la phase huileuse comprend au moins une huile choisie parmi les huiles d'origine végétale ou animale, les huiles minérales et les huiles de synthèse.

14. Composition selon l'une quelconque des revendications précédentes, caractérisée en ce que les particules de gel cubique contiennent au moins un principe actif choisi parmi les principes actifs hydrophiles et lipophiles.

15. Composition selon l'une quelconque des revendications précédentes, caractérisée en ce qu'elle contient au moins un principe actif dans la phase huileuse et/ou dans la phase aqueuse.

16. Composition selon l'une quelconque des revendications précédentes, caractérisée en ce qu'elle contient au moins un agent gélifiant.

17. Composition selon la revendication précédente, caractérisée en ce que l'agent gélifiant est choisi parmi les dérivés de cellulose, les dérivés d'algues, les gommes naturelles et les polymères synthétiques.

18. Composition selon l'une quelconque des revendications précédentes, caractérisée en ce qu'elle constitue une composition cosmétique, dermatologique et/ou pharmaceutique.

19. Composition selon l'une quelconque des revendications précédentes, caractérisée en ce qu'elle se présente sous forme d'une crème, d'une pommade, d'un lait, d'une lotion, d'un sérum, d'une pâte, d'une mousse.

20. Procédé de traitement cosmétique pour le soin et/ou le nettoyage et/ou le conditionnement et/ou le maquillage et/ou la protection de la peau, des fibres kératiniques, du cuir chevelu et/ou des muqueuses, consistant à appliquer sur la peau, les fibres kératiniques, le cuir chevelu et/ou les muqueuses, une composition selon l'une quelconque des revendications précédentes.

21. Utilisation de la composition selon l'une quelconque des revendications 1 à 19 pour la préparation d'une composition thérapeutique destinée au traitement et/ou au soin de la peau, des fibres kératiniques, du cuir chevelu et/ou des muqueuses.

## Patentansprüche

1. Zusammensetzung in Form einer Dispersion, die eine wäßrige Phase und eine Ölphase aufweist, dadurch gekennzeichnet, daß sie ferner Partikel eines kubischen Gels enthält, die aus einem Gemisch von mindestens zwei amphiphilen Verbindungen gebildet werden, wobei eine amphiphile Verbindung befähigt ist, in Gegenwart von Wasser eine lamellare Phase zu bilden und unter Diglycerinisostearat, Diglycerinmonooleat und deren Gemischen ausgewählt ist, und die andere Verbindung befähigt ist, in Gegenwart von Wasser eine inverse hexagonale Phase zu bilden und unter Diglycerin-2-decyltetradecanoat, Diglycerindi/trioleat, 3-N-(2-Decyltetradecyloxycarbonyl)-amino-1,2-propandiol und N-2-Dodecylhexadecyloxycarbonyl-N-methyl-D-glucamin und deren Gemischen ausgewählt ist.

2. Zusammensetzung nach dem vorhergehenden Anspruch, dadurch gekennzeichnet, daß das Gemisch der beiden amphiphilen Verbindungen aus 10 bis 90 Gew.-% der amphiphilen Verbindung, die eine lamellare Phase bilden kann, und 10 bis 90 Gew.-% der amphiphilen Verbindung, die eine inverse hexagonale Phase bilden kann, bezogen auf das Gesamtgewicht des Gemisches, besteht.

3. Zusammensetzung nach Anspruch 1 oder 2, dadurch gekennzeichnet, daß das Gemisch der beiden amphiphilen Verbindungen unter den folgenden Gemischen ausgewählt ist:
- 55 bis 75 Gew.-% Diglycerinisostearat und 25 bis 45 Gew.-% Diglycerin-2-decyltetradecanoat,
- 30 bis 65 Gew.-% Diglycerinisostearat und 35 bis 70 Gew.-% Diglycerindi/trioleat,
- 75 bis 85 Gew.-% Diglycerinisostearat und 15 bis 25 Gew.-% 3-N-(2-Decyltetradecyloxycarbonyl)-amino-1,2-propandiol,
- 55 bis 75 Gew.-% Diglycerinisostearat und 25 bis 45 Gew.-% N-2-Dodecylhexadecyloxycarbonyl-N-methyl-D-glucamin,
- 15 bis 50 Gew.-% Diglycerinmonooleat und 50 bis 85 Gew.-% Diglycerindi/trioleat.

4. Zusammensetzung nach einem der vorhergehenden Ansprüche, dadurch gekennzeichnet, daß sie 0,1 bis 15 Gew.-% amphiphile Verbindungen, die die Partikel der kubischen Phase bilden, bezogen auf das Gesamtgewicht der Zusammensetzung, enthält.

5. Zusammensetzung nach einem der vorhergehenden Ansprüche, dadurch gekennzeichnet, daß sie 2 bis 40 Gew.-% Ölphase, bezogen auf das Gesamtgewicht der Zusammensetzung, enthält.

6. Zusammensetzung nach einem der vorhergehenden Ansprüche, dadurch gekennzeichnet, daß das auf das Gewicht bezogene Verhältnis der amphiphilen Verbindungen und der Ölphase im Bereich von 0,02/1 bis 1/1 und besser noch 0,05/1 bis 0,5/1 liegt.

7. Zusammensetzung nach einem der vorhergehenden Ansprüche, dadurch gekennzeichnet, daß die Partikel des kubischen Gels eine Größe im Bereich von 0,05 bis 1 µm aufweisen.

8. Zusammensetzung nach einem der vorhergehenden Ansprüche, dadurch gekennzeichnet, daß es sich um eine O/W-Emulsion handelt und die Größe der Tröpfchen der dispergierten Ölphase vorzugsweise im Bereich von 0,1 bis 10 µm liegt.

9. Zusammensetzung nach einem der Ansprüche 1 bis 7, dadurch gekennzeichnet, daß es sich um eine W/O-Emulsion handelt.

10. Zusammensetzung nach einem der vorhergehenden Ansprüche, dadurch gekennzeichnet, daß sie ferner mindestens ein stabilisierendes und dispergierendes Mittel enthält, das unter den bei Raumtemperatur wasserlöslichen grenzflächenaktiven Stoffen ausgewählt ist, die eine gesättigte oder ungesättigte, geradkettige oder verzweigte Fettkette mit 8 bis 22 Kohlenstoffatomen aufweisen.

11. Zusammensetzung nach dem vorhergehenden Anspruch, dadurch gekennzeichnet, daß das dispergierende und stabilisierende Mittel unter den polyethoxylierten Alkyl- oder Alkenylestern von Sorbitan ausgewählt ist, die mindestens 20 Ethylenoxideinheiten aufweisen.

12. Zusammensetzung nach einem der vorhergehenden Ansprüche, dadurch gekennzeichnet, daß sie ferner mindestens ein nicht wasserlösliches, ionisches, amphiphiles Lipid enthält.

13. Zusammensetzung nach einem der vorhergehenden Ansprüche, dadurch gekennzeichnet, daß die Ölphase mindestens ein Öl enthält, das unter den Ölen pflanzlichen oder tierischen Ursprungs, Mineralölen und synthetischen Ölen ausgewählt ist.

14. Zusammensetzung nach einem der vorhergehenden Ansprüche, dadurch gekennzeichnet, daß die Partikel eines kubischen Gels mindestens einen Wirkstoff enthalten, der unter den hydrophilen und lipophilen Wirkstoffen ausgewählt ist.

15. Zusammensetzung nach einem der vorhergehenden Ansprüche, dadurch gekennzeichnet, daß sie mindestens einen Wirkstoff in der Ölphase und/oder wäßrigen Phase enthält.

16. Zusammensetzung nach einem der vorhergehenden Ansprüche, dadurch gekennzeichnet, daß sie mindestens einen Gelbildner enthält.

17. Zusammensetzung nach dem vorhergehenden Anspruch, dadurch gekennzeichnet, daß der Gelbildner unter den Cellulosederivaten, Algenderivaten, natürlichen Gummen und synthetischen Polymeren ausgewählt ist.

18. Zusammensetzung nach einem der vorhergehenden Ansprüche, dadurch gekennzeichnet, daß sie eine kosmetische, dermatologische und/oder pharmazeutische Zusammensetzung ist.

19. Zusammensetzung nach einem der vorhergehenden Ansprüche, dadurch gekennzeichnet, daß sie als Creme, Salbe, Milch, Lotion, Serum, Paste oder Schaum vorliegt.

20. Verfahren zur kosmetischen Behandlung, zur Pflege und/oder zur Reinigung und/oder zur Konditionierung und/oder zum Schminken und/oder zum Schutz der Haut, der Keratinfasern, der Kopfhaut und/oder der Schleimhäute, das darin besteht, auf die Haut, die Keratinfasern, die Kopfhaut und/oder die Schleimhäute eine Zusammensetzung nach einem der vorhergehenden Ansprüche aufzutragen.

21. Verwendung der Zusammensetzung nach einem der Ansprüche 1 bis 19 zur Herstellung einer therapeutischen Zusammensetzung, die zur Behandlung und/oder zur Pflege der Haut, der Keratinfasern, der Kopfhaut und/oder der Schleimhäute vorgesehen ist.

## Claims

1. Composition in the form of a dispersion comprising an aqueous phase and an oily phase, characterized in that it also comprises cubic gel particles formed from a mixture of at least two amphiphilic compounds, one of the amphiphilic compounds being capable of forming a lamellar phase in the presence of water and being chosen from diglyceryl isostearate, diglyceryl monooleate and mixtures thereof, and the other being capable of forming an inverse hexagonal phase in the presence of water and being chosen from diglyceryl 2-decyltetradecanoate, diglyceryl di/trioleate, 3-N-(2-decyltetradecyloxycarbonyl)amino-1,2-propanediol and N-2-dodecylhexadecyloxycarbonyl-N-methyl-D-glucamine, and mixtures thereof.

2. Composition according to the preceding claim, characterized in that the mixture of the two amphiphilic compounds consists of from 10% to 90% by weight of the amphiphilic compound capable of forming a lamellar phase and from 10% to 90% by weight of the amphiphilic compound capable of forming an inverse hexagonal phase, relative to the total weight of the mixture.

3. Composition according to Claim 1 or 2, characterized in that the mixture of the two amphiphilic compounds is chosen from the following mixtures:
- 55% to 75% by weight of diglyceryl isostearate and 25% to 45% by weight of diglyceryl 2-decyltetradecanoate;
- 30% to 65% by weight of diglyceryl isostearate and 35% to 70% by weight of diglyceryl di/trioleate;
- 75% to 85% by weight of diglyceryl isostearate and 15% to 25% by weight of 3-N-(2-decyltetradecyloxycarbonyl) amino-1,2-propanediol;
- 55% to 75% by weight of diglyceryl isostearate and 25% to 45% by weight of N-2-dodecylhexadecyloxycarbonyl-N-methyl-D-glucamine;
- 15% to 50% by weight of diglyceryl monooleate and 50% to 85% by weight of diglyceryl di/trioleate.

4. Composition according to any one of the preceding claims, characterized in that it comprises from 0.1% to 15% by weight of amphiphilic compounds constituting the particles of the cubic phase relative to the total weight of the composition.

5. Composition according to any one of the preceding claims, characterized in that it comprises from 2% to 40% by weight of oily phase relative to the total weight of the composition.

6. Composition according to any one of the preceding claims, characterized in that the weight ratio of the amphiphilic compounds and of the oily phase ranges from 0.02/1 to 1/1 and better still from 0.05/1 to 0.5/1.

7. Composition according to any one of the preceding claims, characterized in that the cubic gel particles range from 0.05 µm to 1 µm in size.

8. Composition according to any one of the preceding claims, characterized in that it is an O/W emulsion and in that the size of the droplets of the dispersed oily phase preferably ranges from 0.1 µm to 10 µm.

9. Composition according to any one of Claims 1 to 7, characterized in that it is a W/O emulsion.

10. Composition according to any one of the preceding claims, characterized in that it also contains at least one dispersant and stabilizer chosen from surfactants that are water-soluble at room temperature, containing a saturated or unsaturated, linear or branched fatty chain containing from 8 to 22 carbon atoms.

11. Composition according to the preceding claim, characterized in that the dispersant and stabilizer is chosen from polyoxyethylenated alkyl or alkenyl esters of sorbitan comprising at least 20 ethylene oxide units.

12. Composition according to any one of the preceding claims, characterized in that it also contains at least one water-insoluble ionic amphiphilic lipid.

13. Composition according to any one of the preceding claims, characterized in that the oily phase comprises at least one oil chosen from oils of plant or animal origin, mineral oils and synthetic oils.

14. Composition according to any one of the preceding claims, characterized in that the cubic gel particles contain at least one active principle chosen from hydrophilic and lipophilic active principles.

15. Composition according to any one of the preceding claims, characterized in that it contains at least one active principle in the oily phase and/or in the aqueous phase.

16. Composition according to any one of the preceding claims, characterized in that it contains at least one gelling agent.

17. Composition according to the preceding claim, characterized in that the gelling agent is chosen from cellulose derivatives, algal derivatives, natural gums and synthetic polymers.

18. Composition according to any one of the preceding claims, characterized in that it constitutes a cosmetic, dermatological and/or pharmaceutical composition.

19. Composition according to any one of the preceding claims, characterized in that it is in the form of a cream, an ointment, a milk, a lotion, a serum, a paste or a mousse.

20. Cosmetic treatment process for caring for and/or cleansing and/or conditioning and/or making up and/or protecting the skin, keratin fibres, the scalp and/or mucous membranes, which consists in applying a composition according to any one of the preceding claims to the skin, keratin fibres, the scalp and/or mucous membranes.

21. Use of the composition according to any one of Claims 1 to 19, for the preparation of a therapeutic composition intended for treating and/or caring for the skin, keratin fibres, the scalp and/or mucous membranes.
